# EUROPÄISCHE PATENTSCHRIFT

(11) **EP 0 895 788 B1**
(45) Veröffentlichungstag und Bekanntmachung des Hinweises auf die Patenterteilung: **09.07.2003**
(21) Anmeldenummer: 98114718.4
(22) Anmeldetag: 05.08.1998
(51) Int. Cl.: A61M 15/00, A61M 11/06

(54) **Inhalationstherapiegerät mit einem Ventil zur Begrenzung des Inspirationsflusses**
Inhalation therapy apparatus with valve to limit the inspiration flow
Appareil de thérapie par inhalation avec valve permettant de limiter l'écoulement d'inspiration

(30) Priorität: 06.08.1997 DE 19734022
(43) Veröffentlichungstag der Anmeldung: 10.02.1999
(73) Patentinhaber: PARI GmbH Spezialisten für effektive Inhalation, 82319 Starnberg (DE)
(72) Erfinder: Wunderlich, Eric, 82205 Gilching (DE); Waldner, Robert, 86971 Peiting (DE); Knoch, Martin Dr., 82335 Berg (DE)
(74) Vertreter: HOFFMANN - EITLE

(56) Entgegenhaltungen:
- EP-A- 0 134 847
- EP-A- 0 281 650
- US-A- 3 796 216
- US-A- 5 437 271

## Beschreibung

Die vorliegende Erfindung betrifft Inhalationstherapiegeräte und insbesondere ein Ventil zur Begrenzung des Inspirationsflusses.

Aus EP-B-0 281 650 ist ein Aerosolzerstäuber bekannt, der aus einem im wesentlichen zylindrischen Grundkörper besteht, in dem eine Zerstäuberdüse für die Erzeugung eines Aerosols angeordnet ist und in den ein Zuluftkamin für die Zuführung von Umgebungsluft hineinragt. Die äußere Öffnung des Zuluftkamins ist durch ein Einlaßventil verschlossen, das als Einwegventil ausgebildet ist. Es erlaubt das Zuströmen von Umgebungsluft in das Verneblergehäuse beim Einatmen des Patienten durch ein Mundstück des Aerosolzerstäubers, verhindert aber das Entweichen des Aerosols aus dem Zerstäuberinnenraum während der Atempausen und für den Fall, daß der Patient in den Aerosolzerstäuber hinein ausatmet.Das bekannte Inhalationstherapiegerät ist demnach so gestaltet, daß der Patient ein beliebiges Atemluftvolumen einatmen kann, begrenzt lediglich von dem durch die Gestalt des Zerstäubers gegebenen Strömungswiderstand.

US-A-5437271 zeigt einen Puiververnebler mit einem als steife Wippe ausgeführten Ventilelement, das bei einem bestimmten Durchfluss gegen einen Anschlag stößt um ein komplettes schließen des Kanals zu verhindern.

Ziel einer Inhalationstherapie ist jedoch stets die effektive und nebenwirkungsarme Applikation des in Aerosolform dargebotenen Medikaments in erkrankten Lungenbereichen. Dazu wird in einem Inhalationstherapiegerät ein Aerosol mit geeignetem Tröpfchenspektrum und in geeigneter Menge erzeugt. Dieses Aerosol soll der Patient nach einem bestimmten Atemmuster inhalieren, so daß die Aerosoltröpfchen an den gewünschten Depositionsort in der Lunge gelangen. Bislang war es erforderlich, den Patienten im Hinblick auf dieses Atemmuster und auf eine Optimierung der Atemparameter zu trainieren, was jedoch in vielen Fällen zu erheblichen Schwierigkeiten führte.

Bei diesen Bemühungen stehen sich zwei widersprechende Anforderungen gegenüber. Denn einerseits soll dem Patienten das Einatmen des Aerosols so leicht wie möglich gestaltet werden und andererseits tritt eine Maximierung des Effekts der Inhalationstherpie nur dann ein, wenn ein Aerosol in einer Art und Weise eingeatmet wird, daß eine Deposition an der gewünschten Stelle der Lunge stattfindet. Dieser Vorgang wird durch die Tröpfchengröße, die Aerosolmenge, die Zuluftmenge, den Inspirationsfluß und andere Parameter beeinflußt. Im Hinblick auf den Inspirationsfluß wurde bislang nur versucht, durch Querschnittsreduzierungen (Stenosen) im Inspirationspfad des Inhalationstherapiegerätes eine Begrenzung zu erreichen. Aufgrund des erhöhten Strömungswiderstandes in dem zu realisierenden Flowbereich geht dies jedoch oft zu Lasten der in der Regel ohnehin schon geschwächten Atemleistung des Patienten.

Aerosolphysikalisch wünschenswert ist ein Inspirationsfluß bis maximal 30 l/min, um die Trägheitsabscheidung, die sogenannte Impaktion, der Aerosoltröpfchen in den oberen Atemwegen zu minimieren und um die Depositionswahrscheinlichkeit in der Lunge an dem beabsichtigten Depositionsort zu erhöhen.

Von geringerer Bedeutung ist der Ausatmungsvorgang. Aber auch beim Ausatmen sollte sich der Expirationsfluß in einem bestimmtem Bereich bewegen.

Vor diesem Hintergrund besteht die Aufgabe der Erfindung darin, ein Inhalationstherapiegerät so auszugestalten, daß für den Patienten die Einhaltung aerosolphysikalisch wünschenswerter Bereiche bei der Atmung erleichtert wird, ohne daß bei der Atmung in diesen Bereichen eine zusätzliche Belastung des Patienten auftritt.

Gelöst wird diese Aufgabe durch ein Inhalationstherapiegerät mit einem Vernebelungsraum, in dem mit Hilfe einer Zerstäuberdüse ein Aerosol erzeugt wird, einem Anschlußstutzen, über den das Aerosol aus dem Vernebelungsraum herausgeführt wird, einer Lufteinlaßeinrichtung, durch die Umgebungsluft in den Vernebelungsraum gelangt, und einem Einlaßventil, das zumindest eine Einlaßöffnung und ein die Einlaßöffnung verschließendes Ventilelement aus Silikon oder einem anderen elastischen Material umfaßt und das in der Lufteinlaßeinrichtung derart angeordnet ist, daß ein Unterdruck im Vernebelungsraum das Ventilelement bewegt, um die Einlaßöffnung freizugeben, wobei im Einlaßventil ein Begrenzerelement vorgesehen ist, das die Bewegung des Ventilelements derart beschränkt, daß die Freigabe der Einlaßöffnung nur bis zu einem Schwellwert des Unterdrucks im Vernebelungsraum im wesentlichen proportional zum Unterdruck ist.

In einer besonderen Gestaltung dieser Lösung weist das Begrenzerelement zumindest eine Durchtrittsöffnung auf, die von dem Ventilelement teilweise verschlossen wird, wenn das Ventilelement durch einen den Schwellwert übersteigenden Unterdruck in dem Vernebelungsraum bis an das Begrenzerelement bewegt wird.

Vorteilhaft ist bei dem erfindungsgemäßen Inhalations therapie gerät, das Ventilelement ein kreisförmiges Ventilplättchen, wobei die Durchtrittsöffnung des Begrenzerelements im Bereich der äußeren Kante des Ventilplättchens zumindest abschnittsweise über die Kontur des Ventilplättchens hinausragt.

Von Vorteil ist ferner, wenn die mit dem Ventilplättchen wechselwirkende Kontur der Luftdurchtrittsöffnung des Begrenzerelements wellenförmig oder zick-zack-förmig gestaltet ist.

Für die Anwendung bei der Aerosoltherapie bedeutet die Erfindung, daß das Inhalationstherapiegerät mit einem Begrenzerventil ausgestattet ist, das an die lungenphysiologischen Parameter des Patienten anpaßbar ist und als passiver Schwellwertregler den Inspirationsfluß des Patienten steuert. Durch eine oberhalb eines bestimmten Flow-Schwellwertes stark progressive Druck/Flow-Charakteristik wird erreicht, daß der Patient an dem beim Einatmen verspürten Strömungswiderstand erkennt, daß er außerhalb des aerosolphysikalisch wünschenswerten Bereichs liegt. Er kann daraufhin sein Atemverhalten entsprechend anpassen. Innerhalb des aerosolphysikalisch wünschenswerten Bereiches verspürt der Patient praktisch keine Erhöhung des Strömungswiderstands im Vergleich zu einem Inhalationstherapiegerät ohne das erfindungsgemäße passive Begrenzerventil.

Das im folgenden noch näher anhand eines Ausführungsbeispiels beschriebene Begrenzerventil besitzt ferner den Vorteil, daß es extrem einfach realisiert ist und so die gewünschten Ziele auf kostengünstige Weise erreicht werden.

Im folgenden wird die Erfindung anhand eines Ausführungsbeispiels unter Bezugnahme auf die Figuren genauer erläutert, in denen zeigt:
- Fig. 1: ein Inhalationstherapiegerät mit einem Begrenzerventil gemäß der Erfindung;
- Fig. 2A bis 2C: das Begrenzerventil aus Fig. 1 in verschiedenen Ansichten, und
- Fig. 3: ein Diagramm der Druck/Fluß-Verhältnisse bei einem erfindungsgemäß ausgestalteten Inhalationstherapiegerät.

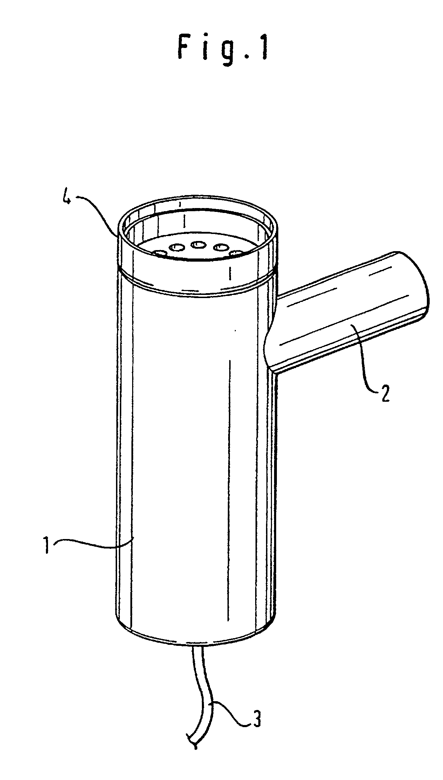

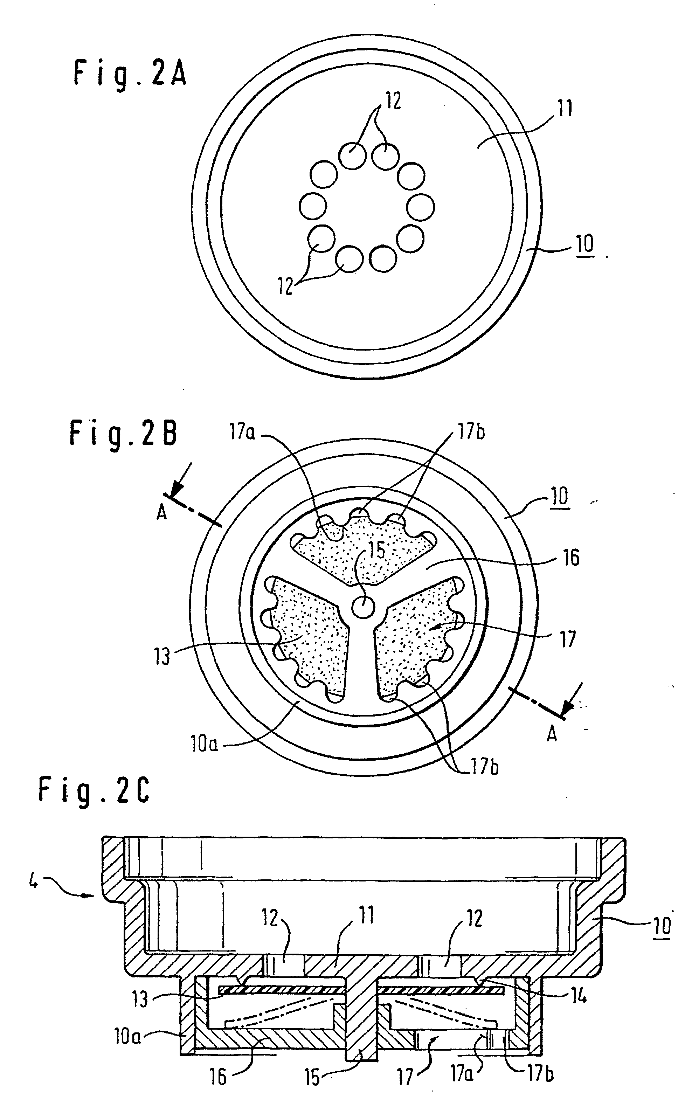

In Fig. 1 ist ein Inhalationstherapiegerät dargestellt, das aus einem zylindrischen Grundkörper 1 und einem daran angeformten Anschlußstutzen 2 besteht. Im Inneren des zylindrischen Grundkörpers 1, d.h. im Verneblungsraum ist eine Zerstäuberdüse (nicht dargestellt, vgl. beispielsweise EP-B-0 281 650) angeordnet, die aus einem in dem Inhalationstherapiegerät bevorrateten Medikament ein Aerosol erzeugt. Dazu wird der Zerstäuberdüse über eine Druckmittelzuleitung 3 komprimierte Luft zugeführt. Am Anschlußstutzen 2 ist üblicherweise ein Mundstück angeordnet, über das der Patient das im Vernebelungsraum erzeugte Aerosol einatmen kann. Vom in Fig. 1 oberen Ende des zylindrischen Grundkörpers 1 her ragt ein zylindrischer Zuluftkamin (nicht dargestellt, vgl. beispielsweise EP-B-0 281 650) in den Vernebelungsraum hinein. Durch diesen Zuluftkamin kann Umgebungsluft in das Innere des Inhalationstherapiegeräts nachströmen, wenn der Patient über das am Anschlußstutzen 2 angesetzte Mundstück das Aerosols einatmet. Die nach außen weisende Öffnung des Zuluftkamins ist verschlossen durch ein passives Begrenzerventil 4, das als Einwegventil ausgestaltet ist und dessen Aufbau im folgenden unter Bezugnahme auf Fig. 2 genauer beschrieben ist.

Fig. 2A zeigt das passive Begrenzerventil 4 von der nach außen gewandten Seite, d.h. von der Seite, die auch in Fig. 1 erkennbar ist. Fig. 2B zeigt das passive Begrenzerventil 4 auf der dem Zuluftkamin zugewandten Seite, die in Fig. 1 nicht sichtbar ist. Fig. 2C zeigt einen Querschnitt durch das passive Begrenzerventil entlang der Linie A-A in Fig. 2B.

In den Fig. 2A bis 2C ist erkennbar, daß das passive Begrenzerventil 4 des hier beschriebenen Ausführungsbeispiels der Erfindung aus einem zylindrischen Grundkörper 10 mit einer in mehreren Stufen gestalteten Mantelfläche und einer senkrecht zur Zylinderachse verlaufenden Verschlußwand 11 besteht. In der Verschlußwand 11 sind mehrere Lufteinlaßöffnungen 12 vorgesehen, die auch als Durchlaßöffnungen bezeichnet werden können und durch die Umgebungsluft in das Innere des Inhalationstherapiegeräts strömen kann. In dem Zylinderabschnitt 10a mit dem kleinsten Außendurchmesser ist ein Ventilelement 13 angeordnet, das die Form eines kreisförmigen Ventilplättchens mit einer mittigen Befestigungsöffnung besitzt. Das Ventilplättchen 13 besteht aus einem elastischen Material, beispielsweise Silikon, mit einer ausreichenden Eigensteifigkeit, die gewährleistet, daß das Ventilplättchen 13 in der nicht ausgelenkten Ruhestellung die Lufteinlaßöffnungen 12 des Begrenzerventils 4 verschließt. Wie in Fig. 2C erkennbar, kann dazu unterstützend auf der dem Ventilplättchen zugewandten Seite der Verschlußwand 11 eine kreisförmig umlaufende Dichtlippe 14 vorgesehen sein. Das Ventilplättchen 13 ist zur Befestigung auf einen sich entlang der Zylinderachse erstreckenden Zapfen 15 auf der dem Innenraum des Inhalationstherapiegerätes zugewandten Seite des Begrenzerventils 4 aufgesteckt.

Durch diesen Aufbau wird folgende Funktion realisiert. Atmet der Patient durch das Inhalationstherapiegerät ein, wird das Ventilplättchen ausgelenkt und erlaubt dabei das Zuströmen von Zuluft durch die Lufeinlaßöffnungen 12 des Begrenzerventils 4. Der sich einstellende Öffnungsquerschnitt ist weitgehend proportional zu dem im Verneblerraum aufgebauten Unterdruck. In Atempausen oder beim Ausatmen des Patienten in das Inhalationstherapiegerät hinein verschließt das Ventilelement 13 die Öffnungen 12.

Erfindungsgemäß ist auf der der Verschlußwand 11 gegenüberliegenden Seite des Ventilplättchens 13 ein Begrenzerelement 16 angeordnet, das die Auslenkung des Ventilplättchens 13 beschränkt. Das Begrenzerelement 16 besitzt die Form einer kreisrunden Scheibe mit mittig angeordneter Befestigungsöffnung. Der Außendurchmesser des Begrenzerelements 16 entspricht im wesentlichen dem Innendurchmesser des Zylinderabschnitts 10a. Das Begrenzerelement 16 ist ebenfalls auf den sich entlang der Zylinderachse erstreckenden Zapfen 15 aufgesteckt, so daß das Ventilplättchen 13 und das Begrenzerelement 16 zueinander ausgerichtet sind. Das Begrenzerelement 16 besitzt Luftdurchtrittsöffnungen 17, die ausreichend groß ausgestaltet sind, um dem Hindurchströmen der angesaugten Luft dann keinen großen Widerstand entgegenzusetzen, wenn das Ventilplättchen 13 nur soweit ausgelenkt wird, daß es noch nicht an dem Begrenzerelement 16 anschlägt. Die Umgebungsluft strömt dann durch die Lufteinlaßöffnungen 12 hindurch und um das Ventilplättchen 13 herum, um anschließend durch die Luftdurchtrittssöffnungen 17 in das Innere des Inhalationstherapiegeräts zu gelangen. Durch die dem Ventilplättchen 13 eigene Rückstellkraft wird ein Strömungsweg mit einem Öffnungquerschnitt geschaffen, der dem Unterdruck im Inneren des Inhalationstherapiegeräts, d.h. der Druckdifferenz über dem Ventil, proportional ist, solange der Patient in dem aerosolphysikalisch wünschenswerten Druck-/Flowbereich atmet.

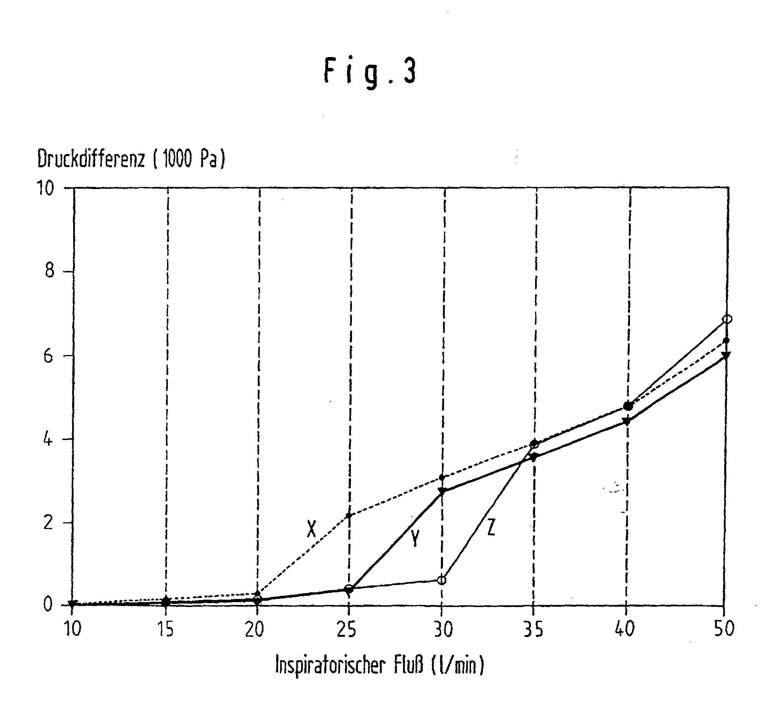

Überschreitet der Unterdruck eine bestimmte Grenze legt sich das Ventilplättchen 13 an die ihm zugewandte Oberfläche des Begrenzerelements 16 an. Dabei werden die Luftdurchtrittsöffnungen 17 des Begrenzerelements nahezu vollständig verschlossen. Aufgrund der Gestaltung der Luftdurchtrittsöffnungen 17 bleiben jedoch Öffnungen für den Luftdurchtritt mit stark reduziertem Querschnitt geöffnet. Die verbleibenden Öffnungen werden im folgenden als Luftdurchtrittsnebenöffnungen bezeichnet; sie können getrennt von den Luftdurchtrittsöffnungen 17 vorgesehen oder als Teil dieser Öffnungen realisiert werden. Der Querschnitt dieser Öffnungen ändert sich nicht mehr, wenn der Unterdruck weiter erhöht wird, d.h. wenn der Patient versucht, stärker einzuatmen. Dies führt zu einem exponentiell ansteigenden Durchströmwiderstand bei weiterer Erhöhung des Unterdrucks, wie dem Diagramm in Fig. 3 (vgl. Verläufe x, y und z) zu entnehmen ist. Letztlich führt dies zu einer Reduzierung des Inspirationsflusses in den gewünschten Sollwertbereich. Dieser Sollwertbereich ist abhängig von der Auslenkung des Ventilplättchens und des Querschnitts der Nebenöffnungen und deshalb in weiten Grenzen einstellbar.

Die Luftdurchtrittsöffnungen 17 des Begrenzerelements 16 sind vorteilhaft so ausgelegt, daß sie eine möglichst große Fläche besitzen und mit den Ventilplättchen 13 ausgerichtet sind. Die entlang des äußeren Randes des Ventilplättchens verlaufende Kontur 17a der Luftdurchtrittsöffnungen 17 ist in dem gezeigten Ausführungsbeispiel so gestaltet, daß auch bei anliegendem Ventilplättchen 13 ein Teil der Luftdurchtrittsöffnungen 17 als Nebenöffnungen 17b unverschlossen bleiben, durch die weiterhin Luft hindurchströmen kann. Diese Nebenöffnungen sind sehr viel kleiner als die Luftdurchlaßöffnungen. Die zuvor angesprochene Kontur der Luftdurchtrittsöffnungen 17 ist beispielsweise wellen- oder zickzackförmig.

In Fig. 2B erkennt man eine wellenförmige Gestaltung der Kontur 17a der Luftdurchtrittsöffnungen 17 in dem der äußeren Kante des Ventilplättchens entsprechenden Bereich. Wenn das Ventilplättchen 13 am Begrenzerelement 16 anliegt, bleiben nur die radial nach außen vortretenden Abschnitte der Luftdurchtrittsöffnungen 17 als Nebenöffnungen 17b unverschlossen, während der weitaus größere Teil der Luftdurchtrittsöffnungen von dem Ventilplättchen 13 verschlossen wird.

Mit der äußeren Kante des Ventilplättchens 13 wirkt somit die entsprechende Kontur 17a der Luftdurchtrittsöffnung 17 des Begrenzerelements 16 zusammen, so daß auch bei anliegendem Ventilplättchen 13 Öffnungen unverschlossen bleiben, durch die Luft hindurchströmen kann. Zwar handelt es sich bei diesen Öffnungen vorzugsweise um Teile der Luftdurchtrittsöffnungen 17, die bei nicht an dem Begrenzerelement anliegendem Ventilplättchen als Ganzes für das Hindurchströmen der Luft zur Verfügung stehen. Bei anliegenden Ventilplättchen stellen sich diese Abschnitte jedoch als Nebenöffnungen dar, durch die weiterhin Luft strömen kann, jedoch aufgrund des verringerten Querschnitts gegen einen erhöhten Strömungswiderstand. Das Zusammenwirken des äußeren Randes des Ventilplättchens mit der korrespondierenden Kontur der Luftdurchlaßöffnung stellt eine besondere Eigenschaft der Erfindung dar. Durch diese Gestaltung wird ein Ventil geschaffen, das der durchströmenden Luft mit steigender Druckdifferenz zunächst proportionale Luftdurchtrittöffnungen mit maximalem Querschnitt und ab einem Schwellwert der Drukdifferenz nur Luftdurchtrittsnebenöffnungen mit deutlich verkleinerter und konstanter Querschnittsfläche zur Verfügung steht.

## Patentansprüche

1. Inhalationstherapiegerät mit
- einem Vernebelungsraum (1), in dem mit Hilfe einer Zerstäuberdüse ein Aerosol erzeugt wird,
- einem Anschlußstutzen (2), über den das Aerosol aus dem Vernebelungsraum herausgeführt wird,
- einer Lufteinlaßeinrichtung, durch die Umgebungsluft in den Vernebelungsraum gelangt, und
- einem Einlaßventils (4), das zumindest eine Einlaßöffnung (12) und ein die Einlaßöffnung verschließendes Ventilelement (13) aus Silikon oder einem anderen elastischen Material umfaßt und das in der Lufteinlaßeinrichtung derart angeordnet ist, daß ein Unterdruck im Vernebelungsraum das Ventilelement bewegt, um die Einlaßöffnung freizugeben,
**dadurch gekennzeichnet,daß**
- im Einlaßventil (4) ein Begrenzerelement (16) vorgesehen ist, das die Bewegung des Ventilelements (13) derart beschränkt, daß die Freigabe der Einlaßöffnung nur bis zu einem Schwellwert des Unterdrucks im Vernebelungsraum im wesentlichen proportional zum Unterdruck ist.

2. Inhalationstherapiegerät nach Anspruch 1, **dadurch gekennzeichnet, daß** das Begrenzerelement (16) zumindest eine Durchtrittsöffnung (17) aufweist, die von dem Ventilelement (13) teilweise verschlossen wird, wenn das Ventilelement (13) durch einen den Schwellwert übersteigenden Unterdruck in dem Vernebelungsraum bis an das Begrenzerelement bewegt wird.

3. Inhalationstherapiegerät nach einem der Ansprüche 1 oder 2, **dadurch gekennzeichnet, daß** das Ventilelement (13) ein kreisförmiges Ventilplättchen ist und daß die Durchtrittsöffnung (17) des Begrenzerelements (16) im Bereich der äußeren Kante des Ventilplättchens zumindest abschnittsweise über die Kontur des Ventilplättchens (13) hinausragt.

4. Inhalationstherapiegerät nach Anspruch 3, **dadurch gekennzeichnet,daß** die mit dem Ventilplättchen wechselwirkende Kontur der Luftdurchtrittsöffnung (17) des Begrenzerelements (16) wellenförmig oder zick-zack-förmig gestaltet ist.

## Claims

1. Inhalation therapy device with
- a nebulisation chamber (1) in which an aerosol is generated by means of an atomiser nozzle,
- a connection piece (2) via which the aerosol is conducted out of the nebulisation chamber,
- an air inlet device through which ambient air passes into the nebulisation chamber, and
- an inlet valve (4) which includes at least one inlet opening (12) and a valve element (13) made of silicone or another resilient material closing the inlet opening and which is arranged in the air inlet device in such a way that a partial pressure in the nebulisation chamber moves the valve element to clear the inlet opening,
**characterised in that**
- in the inlet valve (4) is provided a limiter element (16) which limits the movement of the valve element (13) in such a way that clearing of the inlet opening is essentially proportional to the partial pressure only up to a threshold value of the partial pressure in the nebulisation chamber.

2. Inhalation therapy device according to claim 1, **characterised in that** the limiter element (16) comprises at least one through-opening (17) which is partially closed by the valve element (13) when the valve element (13) is moved as far as the limiter element by a partial pressure in the nebulisation chamber exceeding the threshold value.

3. Inhalation therapy device according to either of claims 1 or 2, **characterised in that** the valve element (13) is a circular valve lamina and **in that** the through-opening (17) of the limiter element (16) in the region of the outer edge of the valve lamina extends in at least one section beyond the contour of the valve lamina (13).

4. Inhalation therapy device according to claim 3, **characterised in that** the contour of the air through-opening (17) of the limiter element (16), which interacts with the valve lamina, is undulating or zigzag-shaped.

## Revendications

1. Appareil de thérapie par inhalation, comprenant :
- une enceinte de nébulisation (1), dans laquelle un aérosol est produit à l'aide d'une buse de pulvérisation,
- une tubulure de raccordement (2), par l'intermédiaire de laquelle l'aérosol est sorti de l'enceinte de nébulisation ,
- un dispositif d'admission d'air, au moyen duquel l'air ambiant pénètre dans l'enceinte de nébulisation, et
- une soupape d'admission (4), comprenant au moins une ouverture d'admission (12) et un élément de soupape ou opercule (13) fermant l'ouverture d'admission, réalisé en silicone ou en un autre matériau élastique et disposé dans le dispositif d'admission d'air, de manière qu'une dépression régnant dans l'enceinte de nébulisation déplace l'élément de soupape pour libérer l'ouverture d'admission,
**caractérisé en ce que**
- dans la soupape d'admission (4) est prévu un élément limiteur (16) limitant le déplacement de l'élément de soupape (13), de manière que la libération de l'ouverture d'admission ne soit que pratiquement proportionnelle à la valeur de la dépression, que jusqu'à une valeur de seuil de la dépression dans l'enceinte de nébulisation.

2. Appareil de thérapie par inhalation selon la revendication 1, **caractérisé en ce que** l'élément limiteur (16) présente au moins une ouverture de passage (17) fermée partiellement par l'élément de soupape (13), lorsque l'élément de soupape (13) est déplacé jusqu'à l'élément limiteur, par une dépression régnant dans l'enceinte de nébulisation et dont la valeur est supérieure à la valeur de seuil.

3. Appareil de thérapie par inhalation selon l'une des revendications 1 ou 2, **caractérisé en ce que** l'élément de soupape (13) est une plaquette de soupape à forme circulaire, et **en ce que** l'ouverture de passage (17) de l'élément limiteur (16), dans la zone du bord extérieur de la plaquette de soupape, dépasse au moins par tronçons hors du contour de la plaquette de soupape (13).

4. Appareil de thérapie par inhalation selon la revendication 3, **caractérisé en ce que** le contour, interagissant avec la plaquette de soupape, de l'ouverture de passage d'air (17) de l'élément limiteur (16) est de forme ondulée ou en zigzag.
